Europäisches Patentamt

European Patent Office

Office européen des brevets

(11) Publication number: **0 308 380**
**A2**

# EUROPEAN PATENT APPLICATION

(21) Application number: **88850287.9**

(22) Date of filing: **31.08.88**

(51) Int. Cl.⁴: **A 61 M 5/315**

(30) Priority: **15.09.87 NO 873863    15.12.87 NO 875234**

(43) Date of publication of application:
**22.03.89 Bulletin 89/12**

(84) Designated Contracting States:
**AT BE CH DE ES FR GB GR IT LI LU NL SE**

(71) Applicant: **ID INTERNATIONAL A/S**
**Drammensveien 175**
**N-0277 Oslo 2  (NO)**

(72) Inventor: **Pettersen, Tor-Erling**
**Steinveien 1**
**N-1453 Bjornemyr  (NO)**

(74) Representative: **Kierkegaard, Lars-Olov et al**
**H. ALBIHNS PATENTBYRA AB P.O. Box 3137**
**S-103 62 Stockholm  (SE)**

(54) **A self-destroying hypodermic syringe.**

**Fig.1.**

(57) A self-destroying hypodermic syringe of the disposable kind comprising a cylinder (1) which carries a cannula (2) at one end. In the cylinder a plunger (3) with a plunger rod (4) is provided, and in connection with said plunger or plunger rod a breaking means (6) is provided to brake after the first time the syringe is used. Breaking means (6) consists of a member (10,11,12) with at least one cam follower (13) cooperating with a cam or coulisse (14) extending obliquely in relation to the syringe axis in another member (15). The cam or coulisse at one end joins an axially extending groove (16) which is open towards one end of member (15). A stop for the cam follower is provided at the cam end opposed to the connection with groove (16). In connection with the plunger rod or the latter and the plunger a freely turnable member (7), e. g. a sphere (8) is provided to run freely in a bearing brass (9).

EP 0 308 380 A2

**Description**

## A self-destroying hypodermic syringe.

The present invention relates to a self-destroying hypodermic syringe comprising a cylinder with a cannula at one end, and a plunger with a plunger rod, of the kind comprising a breaking means in connection with the plunger or plunger rod, which breaking means is broken after the first time the syringe was used.

The invention is primarily of interest in connection with disposable hypodermic syringes. Such syringes are much used both by doctors, in hospital, by diabetics or others who need injections frequently, as well as by dope addicts taking shots. In connection with the latter category of users great problems did arise with reuse of disposable syringes. Diseases communicated by transmission of blood recently showed alarming dissemination due to the fact that dope addicts lend hypodermic syringes to others. This regards hepatite B and AIDS.

Self-destroying hypodermic syringes are known which have a breaking means which is broken after the syringe was used a first time. The previously proposed concepts, however, proved to be too expensive in production or unreliable in use, since it was possible to manipulate the syringe in such a manner that it might be used again.

It is, thus, an object of the present invention to provide a self-destroying hypodermic syringe of the disposable kind which is inexpensive in production and safe in use, i.e. it is destroyed after having been used once, but it may be ventilated without activation of the breaking means.

This is achieved by a self-destroying hypodermic syringe of the kind mentioned above which is characterized by the fact that the breaking means consists of a member with at least one cam follower cooperating with a cam or coulisse in another member which extends obliquely in relation to the syringe axis, and which cam or coulisse is at one end joined with an axially extending groove which is open towards one end of said member, that a stop is provided for the pin at the end of the cam which is opposite to the connection with said groove.

In a preferred embodiment the cam follower or followers are provided near a member which is attached to the plunger rod, via said turnable member, and the member with said cam or coulisses is attached to the plunger.

When an injection is to be given by the aid of a hypodermic syringe the latter must be used after the liquid to be injected is sucked into the syringe by pulling the plunger upwards. Then the plugner is pushed slightly into the syringe cylinder with the tip of the syringe facing upwards, until all air in the syringe is expelled and liquid starts to trickle out of the syringe needle. Then the needle is stabbed into tissue. Then aspiration must take place, i.e. the plunger is slightly retracted to control that the needle is correctly placed. In case of an intravenous injection a little blood should enter into the syringe during retraction of the plunger. In order to be able to carry out said steps when injecting by the aid of a disposable syringe of the above mentioned kind, a preferred embodiment of the invention is characterized by the fact that there are two cam followers provided to face each other at the end of two arms projecting axially from the member, that said member is a cylindrical member which is integrated with the plunger, that the oblique groove extends towards the plunger from the portion of the member facing away from the plunger, that the oblique groove at the end closest to the plunger joins another, oppositely directed oblique groove which at its end far from the plunger joins a further oblique groove which has the same direction as the first groove, that the oblique groove at its end facing the plunger joins a groove which is open towards the free edge of the cylinder, that the first oblique groove where i joins the second groove has less depth than the latter, that the groove where it joins the next groove has less depth than the latter, and that the groove where it joins the next groove has less depth than the latter, so that each groove turns into the subsequent groove, via a step.

Further features of the invention will appear from the dependent claims and from the following disclosure with reference to the drawing which shows two embodiments of a syringe according to the invention.

> Figure 1 is a diagrammatical view of a syringe according to the invention.
> Figure 2 shows an embodiment of a breaking means and the turnable connection between plunger rod and plunger,
> Figure 3 shows a detail of the breaking means in this embodiment,
> Figure 4 is a sectional view along line IV-IV in Figure 3, and
> Figure 5 shows an embodiment which is especially designed to permit ventilation and aspiration of the syringe.

The self-destroying syringe according to the invention comprises a cylinder 1 to the end of which a cannula 2 is attached. In cylinder I a plunger 3 is provided which may be displaced in the cylinder by the aid of a plunger rod with an operating button 5. Plunger rod 4 is connected with plunger 3, via a breaking means generally designated 6, and the plunger rod is connected with the breaking means by the aid of a freely turnable member 7.

Freely turnable member 7 consists of a sphere 8 constructed at the inner end of plunger rod 4. Sphere 8 runs freely in a bearing brass 9 which is provided in a member 10 having two projecting arms 11 and 12 at its free end. At the end of eack arm 11 and 12 a radially inward projecting pin 13 is provided. Each pin 13 cooperates with an oblique groove 14 which is provided in the surface of a cylindrical body 15. Groove 14 starts at the free end of body 15 and extends obliquely towards plunger 3. At its inner end the oblique groove turns into an axially provided groove 16 in body 15 which has an open end 17 at the edge of body 15. In order to simplify insertion of pin 13 in groove 14 a lead-in groove 16 extends from upper edge of body 15.

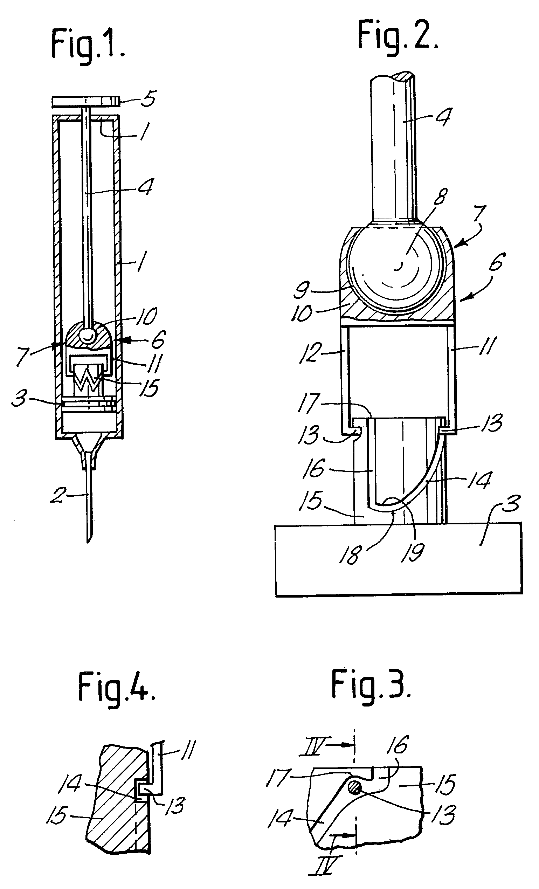

The syringe is mounted in the position as shown in Figures I and 2, i.e. with plugner 3 at the bottom of the syringe and the pins at the top of groove 14, so that pin or pins 13 are in contact with a stop edge 17 at the end of groove 14. The plunger can, thus, be pulled outwards and liquid can enter the syringe through the cannula which is inserted into a liquid container. Since there is friction between cam follower and cam or coulisse, the syringe can be vwnrilated and aspirated without the pin or pins moving along the cam or cams. Then the injection is administered, and the plunge rod is moved downwards. The resistence against forcing liquid in will now be so great that the pins will follow the cams and reach the bottom 18 of oblique groove 14. Plunger 3 now remains on the bottom of the syringe, as shown in Figures 1 and 2, with the difference only, that pins 13 are at the bottom 18 of grooves 14. If efforts are now made to pull the plunger upwards by the aid of plunger rod and operating button 5, pins 13 which are now in the position as indicated by a dashed line, will enter groove 16 and pass out at the open ends of said groove at 17. Due to the freely turnable member 7 it will not be possible to manipulate the syringe to make the pins 13 enter groove 14 by pulling out plunger rod 4. It is, furthermore, ensured that pins 13 will enter groove 16 when plunger rod 4 is pulled out. This is achieved by the aid of an obliquely outwards directed lead-in edge 19 provided across the lower end of groove 14 and having its lowermost point laterally of the lowermost point of groove 14. When plunger rod 4 is pulled out said lead-in 19 will safely guide pin 13 into groove 16.

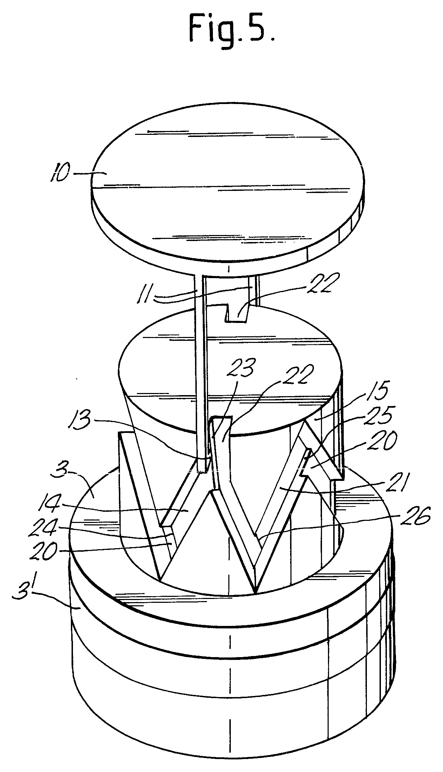

Figure 5 shows the breaking means in a special embodiment permitting ventilation and aspiration of the syringe without breaking the connection between plunger and plunger rod.

Similar to the above embodiment this embodiment comprises two cam followers 13 (only one of which is shown) which are provided at one end each of two flexible arms 11 which are attached to body 10. The cam followers 13 are shaped like bosses facing each other.

In cylindrical member 15 two equal sets of grooves are provided and are arranged in a zig-zag pattern, and are connected at to diametrically opposed points. The zig-zag pattern comprises the above mentioned groove 14 starting at the free edge of member 15 and extending obliquely towards plunger 3. At the end closest to plugner 3 groove 14 is connected with a groove 20 which extends in the opposite direction relative to groove 14. At the end of groove 20 which is closest to the free edge of member 15 it is connected with a groove 21 which extends in the same direction as groove 14. At the end of groove 21 which is closest to plunger 3 this groove is connected with a fourth groove 22 which is open towards the free end of member 15. Groove 14 in the second zig-zag pattern communicates with groove 22 closely to the free edge of member 15. In the area where groove 14 goes into groove 22 in the second zig-zag pattern there is a bar in the shape of a boss or cam 23 in the bottom of the grooves.

When the syringe is to be mounted, bosses or cam followers 13 are introduced into upper end of grooves 22 and are turned axially so as to slide across cams 23 and into groove 14. In this position plunger 3 is mounted in syringe cylinder 1 at the bottom of the latter. The plunger may then be pulled up for filling the syringe. For ventilation plunger 3 is forced inwards by the aid of plunger rod 4 when operation button 5 is depressed. Bosses 3 will then at first slide down along their grooves 14 to the turning point for groove 20. Groove 14 has a decreasing depth from the free end of member 15 and towards the joining point with groove 20, so that groove 20 has a larger depth at the joining point of grooves 14 and 20. A step 24 is, thus, formed between groove 14 and groove 20. Groove 20 also has a decreasing depth towards the point where it joins groove 21. Groove 21 is deeper at the joining point and a step 25 is, thus, formed at the point where groove 20 goes into groove 21. Groove 21 also has a decreasing depth towards groove 22, so that a step 26 is formed between grooves 21 and 22. When the hypodermic syringe is ventilated and bosses 13 are moved down in a groove 14 each they will slide across the step edge on step 24 and down into groove 20. When the plunger rod is then pulled up bosses 13 will follow groove 20 and enter groove 21 by sliding across the edges of steps 25. With the bosses 13 at the joining point between grooves 20 and 21 aspiration may be carried out, i.e. the plunger may be pushed slightly backwards. After aspiration the injection is given and the plunger rod is, thus, forced inwards. Bosses 13 will follow groove 21 and pass across the edge of steps 26. At the joining point between grooves 21 and 22, injection may be carried out by the fact that bosses 13 are in contact with the walls in grooves 21 and 22 in the angle formed between said grooves close to plunger 3. The plunger is then forced all the way in and the injection is administered. If efforts are then made to fill the syringe once more by pulling the plunger rod back, bosses 13 will follow grooves 22 and run freely out of body 15. The connection between plunger rod 4 and plunger 3 is, thus, broken. It is impossible to reconnect plunger rod and plunger by manipulation of the syringe, since plunger rod 4 is connected with plunger 3, via the freely turnable connection 7. The plunger rod with the turnable connection, the breaking means and the plunger cannot be pulled out of the syringe without destroying it. This may be achieved in several manners, e.g. as shown. in Figure 1, where a top 1' is provided to be integrated with the syringe. In syringes where plunger rod 4 has a cruciform cross section with the same dimension as the inner void of the syringe, a top may be provided to comprise two or more tongues which engage in the space between arms in the cruciform cross section.

The invention is not limited to the above disclosed embodiments shown in the drawing. The cam followers may e.g. be provided on the plunger and the cams in connection with the plunger rod. What is essential in this invention is that a cam is activated to move in a radial direction when the plunger rod is pushed in the first time, so that the cam is advanced to the opening of an axial groove which opens outwards.

## Claims

1. A self-destroying hypodermic syringe of the disposable kind, comprising a cylinder (1) which has a cannula (2) provided at one end, with a plunger (3) and a plunger rod (4) provided in the cylinder, and with a breaking means (6) associated with plunger or plunger rod to be broken after the first use of the syringe, **characterized in** that breaking means (6) consists of a member (10) with at least one cam follower (13) cooperating with a cam or coulisse (14) in another member (15), which cam or coulisse extends obliquely in relation to the syringe axis and which cam or coulisse (14) at one end join an axially extending cam or coulisse (16) which opens towards one end of member (15), that a stop (17) for the cam follower (13) is provided at the end of cam (14) opposite its joint with coulisse (16).

2. A hypodermic syringe as defined in claim l, **characterized in** that the cam follower (13) or cam followers are arranged on a member (10) which is attached to the plunger rod, via said turnable member (7), and that member (15) with cam or coulisse (14) is attached to the plunger.

3. A hypodermic syringe as defined in claim 1 or 2, **characterized in** that the cam or coulisse consists of the edge or side of an oblique groove (14) in the surface of a cylindrical member (15) which is attached to the top of the plunger.

4. A self-destroying hypodermic syringe of the disposable kind, comprising a cylinder (1) which has a cannula (2) at one cnd, with a plunger (3) and a plunger rod (4) provided in the cylinder, and with a breaking means (6) associated with plunger or plunger rod, to be broken after the first use of the syringe, **characterized in** that there are two cam followers (13) facing each other and each being provided at the end of one of two arms (11) projecting axially from member (10), that member (15) consists of a cylindrical body (15) which is integrated with plunger (3), that the oblique groove (14) extends from the plunger from the portion of body (15) facing away from plunger (3), that the oblique groove (14) at the end closest to the plunger joins a second oppositely directed oblique groove (20) which at its end far from the plunger joins a further oblique groove (21) extending in the same direction as the first groove (14), that oblique groove (20) at its end facing the plunger joins a groove (22) which opens towards the free edge of cylindrical body (15), that the first oblique groove (14) where it joins the second groove (20) has less depth than the latter, that groove (20) where it joins groove (21) has less depth than the latter, and that groove (21) where it joins groove (22) has less depth than the latter so that each groove (14, 20, 21) passes into the subsequent groove across a step.

5. A hypodermic syringe as defined in one or several of the preceding claims, **characterized in** that there is such friction between cam follower (13) and cam (14) that the syringe may be ventilated and activated without the cam follower moving along the cam.

6. A self-destroying hypodermic syringe as defined in claim 4, **characterized in** that grooves (14, 20, 21) have a decreasing depth towards their subsequent groove.

7. A self-destroying hypodermic syringe as defined in claim 4 or 6, **characterized in** that grooves (14, 20, 21, 22) form a zig-zag pattern on the surface of body (15).

8. A self-destroying hypodermic syringe as defined in claim 7, **characterized in** that groove (14) in one zig-zag pattern joins groove (22) which is open towards the end of body (15) in the other zig-zag pattern closely to the open end of the latter.

9. A self-destroying hypodermic syringe as defined in claim (8), **characterized in** that a boss or a transverse cam is arranged in the bottom of the grooves at the joining point of groove (14) and groove (22) in the second zig-zag pattern.

10. A self-destroying hypodermic syringe as defined in claims 4 and 9, **characterized in** that the two arms (11) which carry the cam followers (13) are manufactured from a resilient material, so that the cam followers when introduced into one groove (22) each which open towards the end of body (15) may be displaced from said groove into the oblique groove (14) of the second zig-zag pattern by an axially rotating movement of a certain force.

11. A self-destroying hypodermic syringe, as defined in claim 1, **characterized in** that a freely turnable member is provided in connection with the plunger rod or between the latter and the plunger.

# Fig.1.

# Fig.2.

# Fig.4.

# Fig.3.

# Fig.5.